# EUROPEAN PATENT SPECIFICATION

(11) **EP 0 335 400 B1**
(45) Date of publication and mention of the grant of the patent: **08.06.1994**
(21) Application number: 89105639.2
(22) Date of filing: 30.03.1989
(51) Int. Cl.: C12N 15/18, C07H 21/04, C12N 1/20, C12P 21/02

(54) **Processes for production of human epidermal growth factor by genetic engineering**
Verfahren zur Herstellung von Epidermal-Wachstumsfaktor durch genetische Transformation
Procédé de production du facteur de croissance épidermique par génie génétique

(30) Priority: 30.03.1988 JP 74382/88; 30.03.1988 JP 74383/88
(43) Date of publication of application: 04.10.1989
(73) Proprietor: HITACHI, LTD., Chiyoda-ku, Tokyo 100 (JP); Hitachi Chemical Co., Ltd., Shinjuku-ku, Tokyo 160 (JP)
(72) Inventor: Shimizu, Norio, Kokubunji-shi (JP); Harada, Yoshinori, Hachioji-shi (JP); Fukuzono, Shinichi, Hachioji-shi (JP); Fujimori, Kiyoshi, Tokyo (JP)
(74) Representative: Strehl Schübel-Hopf Groening & Partner

(56) References cited:
- EP-A- 0 036 776
- EP-A- 0 089 626
- EP-A- 0 147 178
- EP-A- 0 177 915
- WO-A-88/07085
- GB-A- 2 172 890
- CHEMICAL ABSTRACTS, vol. 102, no. 3, January 1985, page 191, abstract no.18893c, Columbus, Ohio, US
- NUCLEIC ACIDS RESEARCH, vol. 9, no. 24, pages 6647-6668; C. YANOFSKY et al.:"The complete nuleotide sequence of the tryptophan operon of Escherichia coli"

## Description

### BACKGROUND OF THE INVENTION

### 1. FIELD OF THE INVENTION

The present invention relates to processes for production of human epidermal growth factor (hereinafter simply referred to as "hEGF") by genetic engineering and more particularly, to novel DNA sequence comprising a chemically synthesized gene coding for hEGF and a part of genes in tryptophan operon, a plasmid containing the DNA sequence, E. coli bearing the plasmid and processes for production of hEGF using them.

### 2. RELATED ARTS

hEGF is considered to be identical with urogastrone and is a polypeptide composed of 53 amino acids which are produced in human duodenum, salivary gland, etc. [H. Gregory and B.M. Preston: Int. J. Peptide Protein Res., 9, 107-118 (1977)]. This polypeptide has the both effects of inhibiting secretion of gastric juice and accelerating the growth of epithelial cells. For this reason, it is likely that hEGF would be applicable to treatment of gastric ulcer or wounds. hEGF is contained in urine generally at a concentration of 25 to 250 ng/ml and has thus been hitherto prepared from urine by purifying the same. However, this purification method is disadvantageous in that the yield is poor.

Recently, attempts have been made to produce hEGF by E. coli using genetic engineering technique. These techniques are described, for example, in Japanese Patent Application KOKAI (Laid-Open) Nos. 57-122096, 60-28994, 61-15691 and 61-88881. In these techniques which comprises incorporating a chemically synthesized hEGF gene into expression vector and culturing E. coli bearing the vector, there should be taken into account (1) DNA sequence coding for the chemically synthesized hEGF, (2) expression vector having a high expression efficiency, (3) conditions for culturing E. coli bearing the expression vector and the like.

In the prior art techniques described above, sufficient consideration has not been made to arrange the DNA sequence coding for hEGF so as to achieve a high translation efficiency and to utilize an expression vector having a high expression efficiency. Therefore, the prior art techniques involve problems that it was difficult to produce hEGF by recombinant E. coli readily in large quantities.

### SUMMARY OF THE INVENTION

Accordingly, an object of the present invention is to provide processes for production of hEGF by genetic engineering which comprises chemically synthesized hEGF gene possessing a high efficiency of translation into hEGF polypeptide, inserting the chemically synthesized hEGF gene into expression vector stable in E. coli and possessing a high expression efficiency, transforming E. coli with the vector and then culturing E. coli bearing the expression vector, thereby producing hEGF efficiently.

Another object of the present invention is to provide a novel DNA sequence comprising the chemically synthesized gene coding for hEGF and a part of genes in tryptophan operon, a plasmid containing such a DNA sequence and recombinant E. coli bearing the plasmid.

A first aspect of the present invention relates to a process for production of human epidermal growth factor by genetic engineering which comprises:
culturing recombinant E. coli bearing a plasmid vector containing a recombinant DNA sequence comprising a tryptophan regulatory gene, a gene coding for the N-terminal of trpE polypeptide in tryptophan operon downstream said regulatory gene, and a gene represented by the following formula (I) coding for 53 amino acids of human epidermal growth factor ligated with said gene coding for the N-terminal through a linker; and
recovering the human epidermal growth factor thus expressed;
and relates to said recombinant DNA sequence and said plasmid vector as well as said recombinant E. coli, used for the process.

### Formula (I):

A A C T C C G A C T C T
G A A T G C C C G C T G T C C C A C G A T
G G T T A C T G C C T G C A C G A C G G C

G T T T G T A T G T A T A T C G A G G C G

C T G G A C A A A T A T G C G T G T A A C

T G T G T A G T A G G C T A C A T C G G C

G A A C G C T G C C A G T A C C G T G A C

C T G A A A T G G T G G G A A C T G C G C

A second aspect of the present invention relates to a process for production of human epidermal growth factor by genetic engineering which comprises:
culturing recombinant E. coli bearing a plasmid vector containing a recombinant DNA sequence comprising a tryptophan regulatory gene, a gene coding for the N-terminal of trpL polypeptide in tryptophan operon downstream said regulatory gene, and a gene represented by formula (I) coding for 53 amino acids of human epidermal growth factor ligated with said gene coding for the N-terminal through a linker; and
recovering the human epidermal growth factor thus expressed;
and relates to said recombinant DNA sequence and said plasmid vector as well as said recombinant E. coli, used for the process.

According to the first and second processes described above, hEGF is expressed as a fused protein.

A third aspect of the present invention relates to a process for production of human epidermal growth factor by genetic engineering which comprises:
culturing recombinant E. coli bearing a plasmid vector containing a recombinant DNA sequence comprising:
a tryptophan regulatory gene,
a DNA sequence for regulating translation of trpL polypeptide in tryptophan operon downstream said tryptophan regulator gene, and
a gene of formula (I) coding for 53 amino acids of human epidermal growth factor immediately downstream a genetic codon coding for methionine at the N-terminal of trpL polypeptide; and
recovering the human epidermal growth factor thus expressed;
and relates to said recombinant DNA sequence and said plasmid vector as well as said recombinant E. coli, used for the process.

A fourth aspect of the present invention relates to a process for production of human epidermal growth factor by genetic engineering which comprises:
culturing recombinant E. coli bearing a plasmid vector containing a recombinant DNA sequence comprising:
a tryptophan regulatory gene,
a trpL polypeptide structural gene in tryptophan operon downstream said tryptophan regulatory gene,
a DNA sequence for regulating translation of trpE polypeptide, and
a gene of formula (I) coding for 53 amino acids of human epidermal growth factor immediately downstream a genetic codon coding for methionine at the N-terminal of trpE polypeptide; and
recovering the human epidermal growth factor. thus expressed;
and relates to said recombinant DNA sequence and said plasmid vector as well as said recombinant E. coli, used for the process.

A fifth aspect of the present invention relates to a process for production of human epidermal growth factor by genetic engineering which comprises:
culturing recombinant E. coli bearing a plasmid vector containing a recombinant DNA sequence comprising:
a tryptophan regulatory gene,
a DNA sequence for regulating translation of trpE polypeptide in tryptophan operon downstream said tryptophan regulatory gene, and
a gene of formula (I) coding for 53 amino acids of human epidermal growth factor immediately downstream a genetic codon coding for methionine at the N-terminal of trpE polypeptide; and
recovering the human epidermal growth factor thus expressed;
and relates to said recombinant DNA sequence and said plasmid vector as well as said recombinant E. coli, used for the process.

In the third to fifth processes described above, hEGF is expressed as matured protein.

### BRIEF DESCRIPTION OF THE DRAWINGS

Fig. 1 shows the entire DNA sequences of hEGF gene synthesized through organic chemistry technique and linker regions upstream and downstream the hEGF gene and the amino acid sequence corresponding to hEGF, wherein A, C, G and T represent adenine nucleotide, cytosine nucleotide, guanine nucleotide and thymine nucleotide, respectively.

Fig. 2 shows DNA sequence comprising promoter/operator (trp P/O) in tryptophan operon and 5'-terminal of trpL leader peptide gene in expression vector pTRL2, wherein PB and SD represent Pribnow box and Shine-Dalgarno sequence, respectively.

Fig. 3 shows DNA sequence comprising promoter/operator (trp P/O) in tryptophan operon, trpL structural gene and 5'-terminal of trpE structural gene in expression vector pTRE2.

Fig. 4 shows the entire DNA sequence of the synthesized hEGF structural gene represented by dividing it into 28 oligonucleotide blocks.

Fig. 5 shows a flow chart for synthesis of oligonucleotide.

Fig. 6 shows the procedure of ligating the oligonucleotide blocks.

Fig. 7 shows the procedure for constructing plasmid pBREGF, wherein Ap^{r} and Tc^{r} represent ampicillinresistant gene and tetracycline-resistant gene, respectively.

Fig. 8 shows the structure of DNA fragment used for constructing plasmid pTRLBT2.

Fig. 9 shows the structure of DNA fragment used for constructing plasmid pTREBT2.

Fig. 10 shows the procedure for constructing plasmid pTRLBT1.

Fig. 11 shows the procedure for constructing plasmid pTREBT1.

Fig. 12 shows the procedure for constructing plasmid pTRLBT2.

Fig. 13 shows the procedure for constructing plasmid pTREBT2.

### DETAILED DESCRIPTION OF THE PREFERRED EMBODIMENTS

### Chemically synthesized gene coding for hEGF:

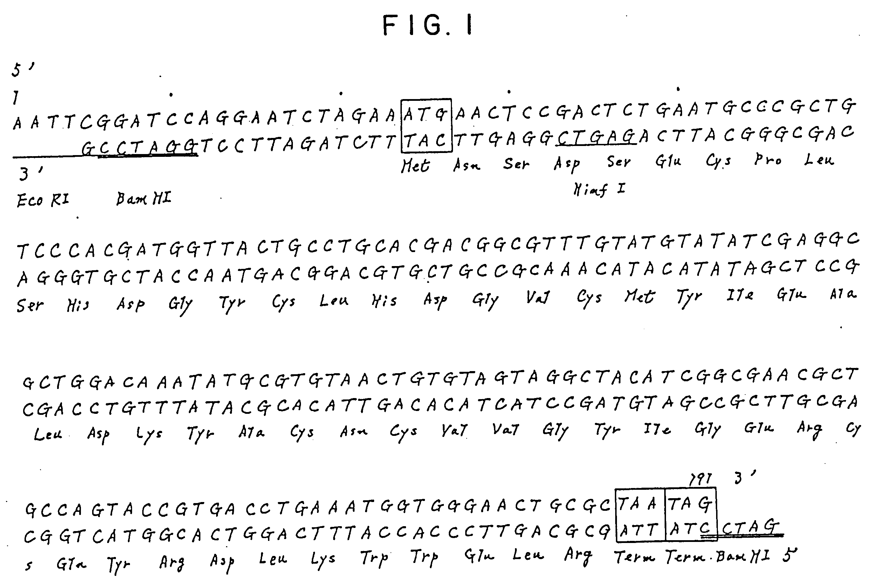

In order to obtain structural gene carrying thereon amino acid sequence information of hEGF composed of 53 amino acids, consideration is taken as to whether, among possible several DNA sequences deduced from the amino acid sequence of hEGF, genetic codon used therein has a high frequency of existence in E. coli, as to whether the structure causes wrong ligation only with difficulty during the course of constructing the entire gene from the organic chemically synthesized gene fragments, and further as to whether reduction in translation efficiency might be caused during the course of translation on ribosome since mRNA transcribed from DNA tends to form a secondary structure such as a hairpin arrangement, etc. Thus, one DNA sequence which was considered to be most pertinent was chosen and synthesized by the technique of organic chemistry. The organic chemically synthesized hEGF-coding sequence is shown in Fig. 1.

ATG (corresponding to formylmethionine) which is a translation initiation codon is provided immediately upstream the DNA sequence corresponding to 53 amino acids of hEGF and the recognition site of restriction enzyme Eco RI is provided upstream ATG as a linker for ligating with plasmid pBR322 [Bolivar et al., Gene 2, 95 (1977)]. On the other hand, both TAA and TAG which are translation termination codons are ligated immediately downstream the DNA sequence. For ligating with plasmid pBR322, the recognition site of Bam HI is further provided.

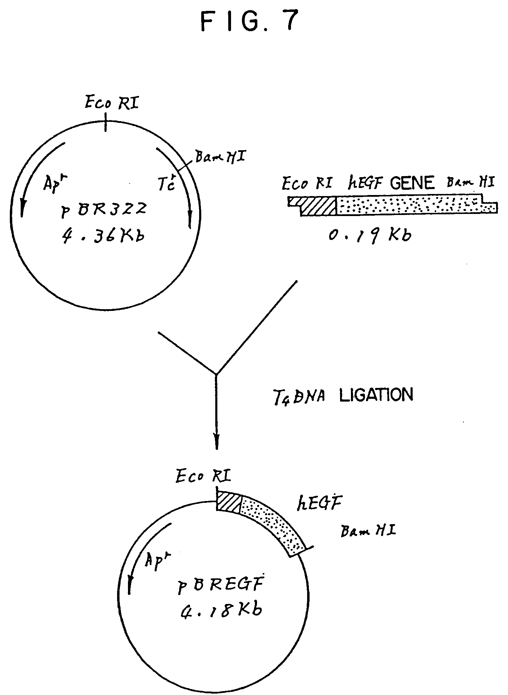

The DNA sequence containing the thus chemically synthesized hEGF structural gene is inserted into the cleavage site provided by treating plasmid pBR322 with restriction enzymes Eco RI and Bam HI to obtain plasmid pBREGF (4.8 killo base pairs (Kb), Fig. 7). E. coli is transformed with this plasmid pBREGF to make it possible to give large quantities of hEGF structural gene stably.

### Plasmid vectors pTREBT1 and pTRLBT1:

Plasmid vectors pTREBT1 and pTRLBT1 are capable of expressing hEGF as fused protein and are used in the first and second aspects of the present invention described above. These plasmid vectors are constructed as follows.

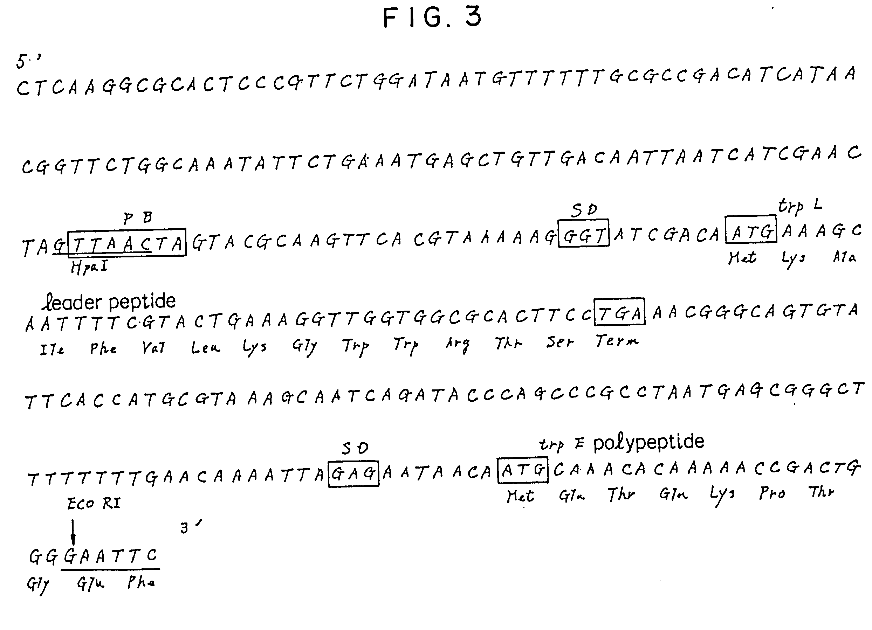

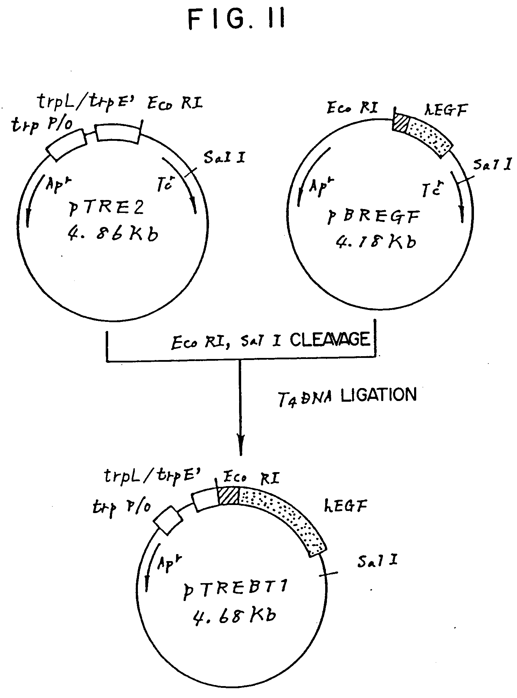

About 500 bp (base pairs) DNA fragment containing promoter/operator which is tryptophan regulatory gene in trp operon derived from E. coli, trpL leader peptide gene and 5'-terminal of trpE (anthranilate synthetase) gene is inserted into the Eco RI site in pBR322 plasmid to give expression vector pTRE2 bearing promoter/operator (P/O) which is a tryptophan regulatory gene and a gene coding for the N-terminal of trpE polypeptide (Fig. 11). In vector pTRE2, the trp promoter is orientated in the same way as Tc^{r} (tetracycline-resistant gene) in pBR322. The vector pTRE2 is modified in such a way that the Eco RI site upstream the trp promoter is filled up with DNA polymerase I to cause deletion and that the unique Eco RI site is present downstream the promoter. In Fig. 3, there is shown DNA sequence comprising trp promoter/operator, trpL structural gene and 5'-terminal of trpE structural gene in expression vector pTRE2. By inserting a foreign gene into the Eco RI site in the trpE polypeptide gene, the foreign gene can be expressed as a protein fused with 8 amino acids at the N-terminal of trpE polypeptide.

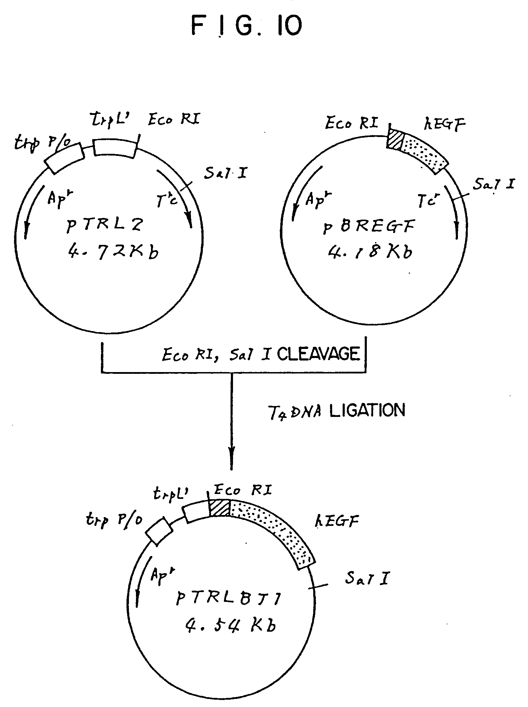

After deleting the restriction enzyme Rsa I site at the 5'-terminal of trpL peptide gene and the entire sequence downstream said site in vector pTRE2, the Rsa I site is modified into the Eco RI site to construct expression vector pTRL2 (Fig. 10). The expression vector pTRL2 contains trp promoter/operator which is a tryptophan regulatory gene, and the gene coding for the N-terminal of trpL polypeptide.

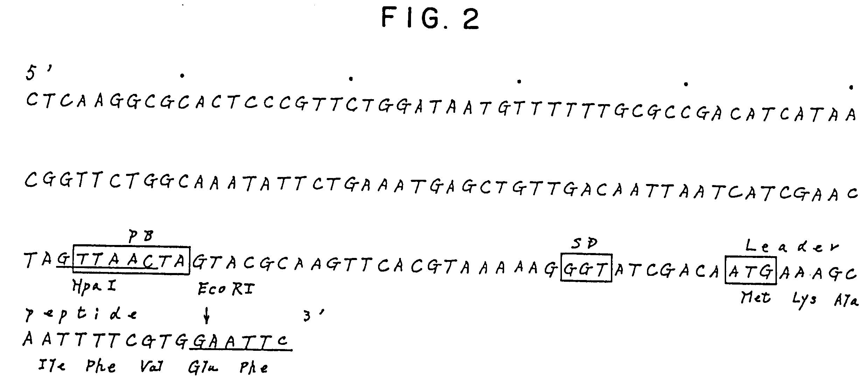

In Fig. 2, there is shown DNA sequence comprising trp promoter/operator and 5'-terminal of trpL peptide gene in expression vector pTRL2. By inserting a foreign gene into the Eco RI site in trpL polypeptide gene, the foreign gene can be expressed as a protein fused with 6 amino acids at the N-terminal of trpL polypeptide.

By inserting the DNA sequence comprising the hEGF gene shown in Fig. 1 into the Eco RI sites in the aforesaid expression vectors pTRE2 and pTRL2, plasmid vectors pTREBT1 (Fig. 11) and pTRLBT1 (Fig. 10) capable of expressing hEGF gene can be constructed, respectively.

Plasmid vector pTREBT1 contains the DNA sequence obtained by ligating the following DNA sequence with the hEGF structural gene: 
A T G C A A A C A C A A A A A C C G A C T
G G G G A A T T C G G A T C C A G G A A T
C T A G A A A T G
Plasmid vector pTRLBT1 contains the DNA sequence obtained by ligating the following DNA sequence with the hEGF structural gene: 
A T G A A A G C A A T T T C G T G G A A
T T C G G A T C C A G G A A T C T A G A A
A T G
The thus constructed vectors pTREBT1 and pTRLBT1 express hEGF as fused proteins to which 17 and 15 amino acids including the amino acids translated from the linkers are bound to the N-terminal of hEGF polypeptide, respectively. In order to obtain matured hEGF free from any superfluous amino acids, there may be adopted a method in which the superfluous amino acids are removed by treating with CNBr (cyanogen bromide) to cleave at the site of methionine (Met). However, since the 21st amino acid within hEGF is Met, this site might also be cleaved but it is known that this Met is cleaved only with difficulty by treating with CNBr [H. Gregory and B.M. Preston: Int. J. Peptide Protein Res., 9, 107-118 (1977)]. Therefore, it is possible to obtain matured hEGF under appropriately selected conditions for the treatment.

### Plasmid vectors pTREBT2 and pTRLBT2:

Plasmid vectors pTRLBT2 and pTREBT2 are capable of expressing hEGF as matured protein and are used in the third and fourth aspects of the present invention described above. These plasmid vectors are constructed as follows.

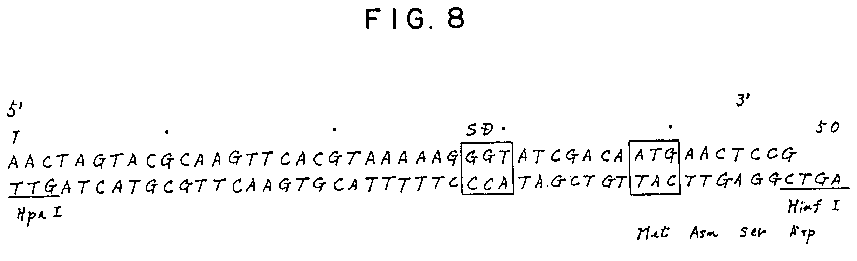

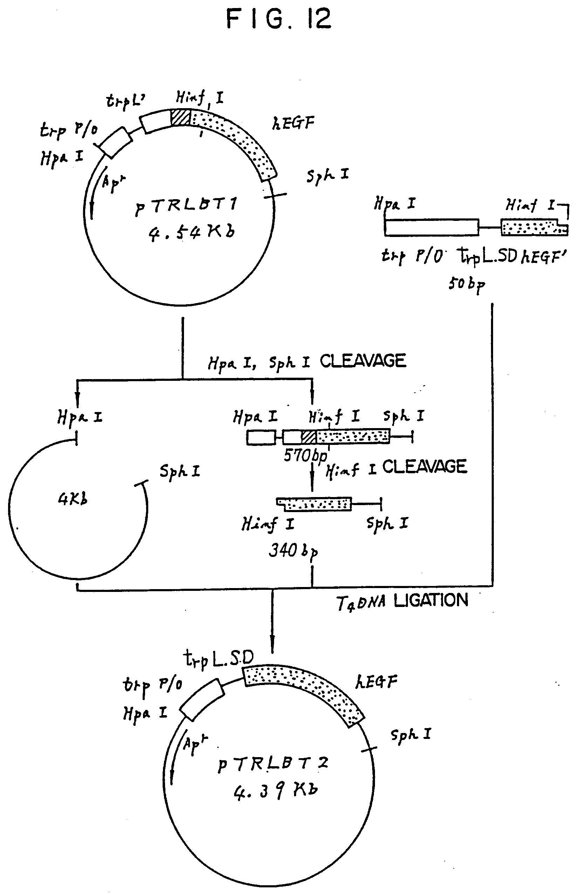

Plasmid vector pTRLBT2 is constructed utilizing trp promoter/operator and Shine-Dalgarno (SD) sequence which is a translation regulatory region of trpL polypeptide in plasmid vector pTRLBT1. That is, pTRLBT2 is constructed in such a manner to position trp promoter/operator, SD sequence of trpL downstream the promoter/operator, the genetic codon coding for methionine (i.e., translation initiation codon) at the N-terminal of trpL polypeptide immediately downstream the SD sequence and then hEGF structural gene directly ligated with the genetic codon. Practically, as shown in Fig. 12, the DNA sequence (Fig. 8) comprising trp promoter/operator, SD sequence, ATG and a part of hEGF structural gene is chemically synthesized, and utilizing this DNA sequence, plasmid vector pTRLBT2 may be constructed.

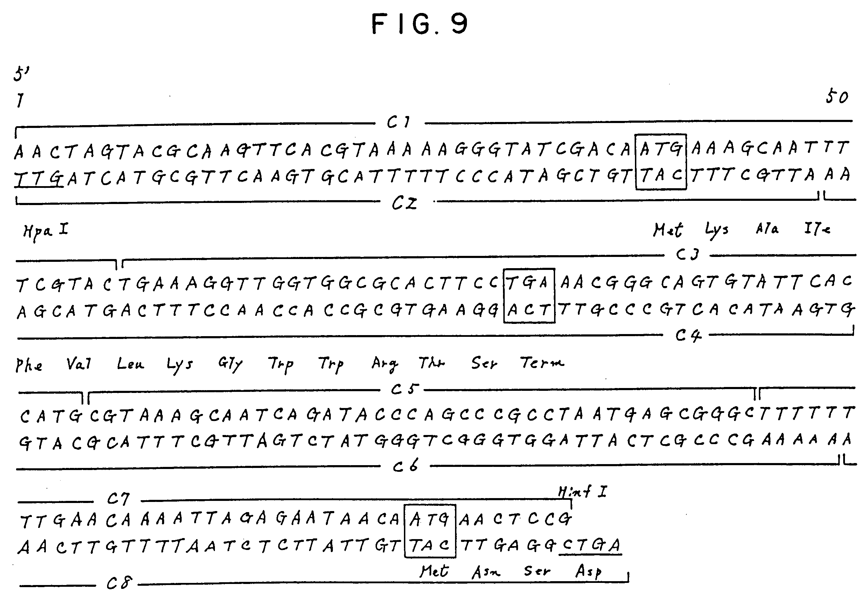

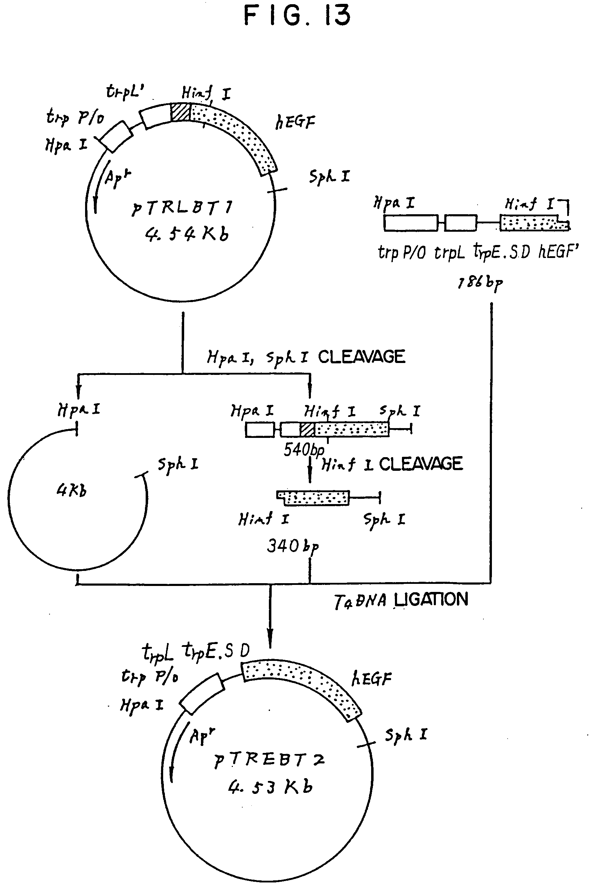

Plasmid vector pTRLBT2 contains the DNA sequence obtained by ligating the following DNA sequence with the hEGF structural gene: 
T T A A C T A G T A C G C A A G T T C A C
G T A A A A A G G G T A T C G A C A A T G
Plasmid vector pTREBT2 is constructed utilizing trp promoter/operator, trpL polypeptide structural gene and SD sequence of trpE in plasmid vector pTREBT1. Practically, as shown in Fig. 13, DNA sequence (Fig. 9) comprising trp promoter/operator, trpL polypeptide structural gene, SD sequence of trpE, ATG and a part of hEGF structural gene is chemically synthesized, and utilizing this DNA sequence, plasmid vector pTRLBT2 may be constructed.

Plasmid vector pTREBT2 contains the DNA sequence obtained by ligating the following DNA sequence with the hEGF structural gene: 
TTAACTAGTACGCAAGTTCACGTAAAAAGGGTATCGACAA
TGAAAGCAATTTTCGTACTGAAAGGTTGGTGGCGCACTTC
CTGAAACGGGCAGTGTATTCACCATGCGTAAAGCAATCAG
ATACCCAGCCCGCCTAATGAGCGGGCTTTTTTTTGAACAA
AATTAAGAGAATAACAATG
Furthermore, a plasmid containing DNA sequence comprising, in this sequence, trp promoter/operator, SD sequence of trpE, ATG and hEGF structural gene can also be constructed as described above. This plasmid vector can be used in the fifth aspect described above. Specifically, the plasmid vector may contain the DNA sequence obtained by ligating the following DNA sequence with the hEGF structural gene:
A G A G A A T A A C A A T G
The plasmid vectors described above can all express matured hEGF polypeptide to which no superfluous amino acids are bonded.

Expression of hEGF polypeptide in E. coli:

Using plasmid vectors pTREBT1, pTRLBT1, pTREBT2, pTRLBT2, etc. described above, E. coli is transformed according to the conventional methods [Maniatis et al., Molecular cloning, 249 (1982)] to give recombinant E. coli bearing these plasmid vectors.

The recombinant E. coli is cultured in a suitable medium, whereby hEGF polypeptide is expressed. Upon culture, it is preferred that the culture be carried out in the presence of 3-β-indolylacrylic acid (IA) as an expression-inducing substance. It is also preferred that the culture be carried out in tryptophan-free medium.

The thus expressed hEGF protein is recovered in a conventional manner to give the objective hEGF polypeptide.

The hEGF structural gene and recombinant DNA sequence as well as plasmid vectors bearing the same described hereinabove are not limited to those specifically disclosed herein but those equivalent to these hEGF structural gene, recombinant DNA sequence and plasmid vectors that can similarly function are within the scope of the present invention.

Hereinafter the present invention is described more specifically by referring to the examples but is not intended to be limited thereto.

### Example 1

Design of DNA sequence of hEGF structural gene and its chemical synthesis are described below.

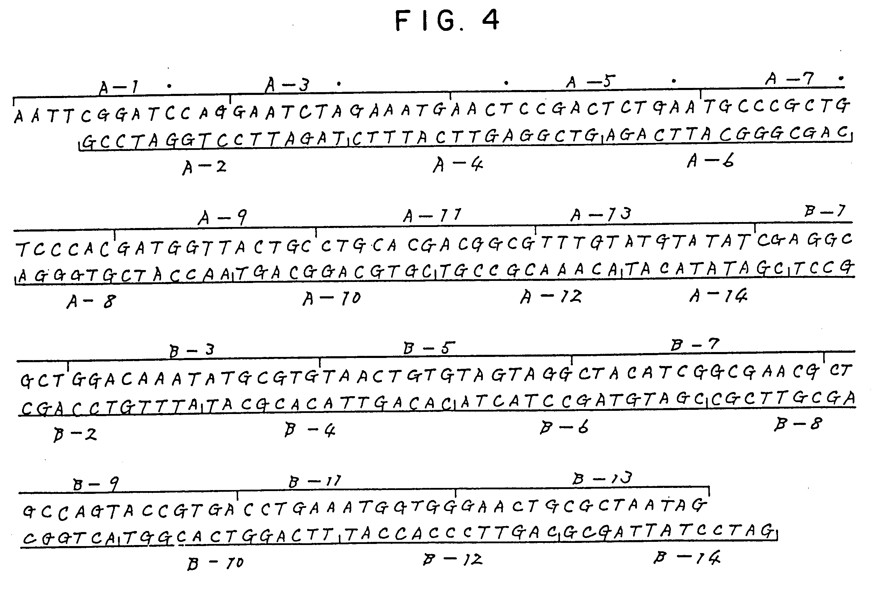

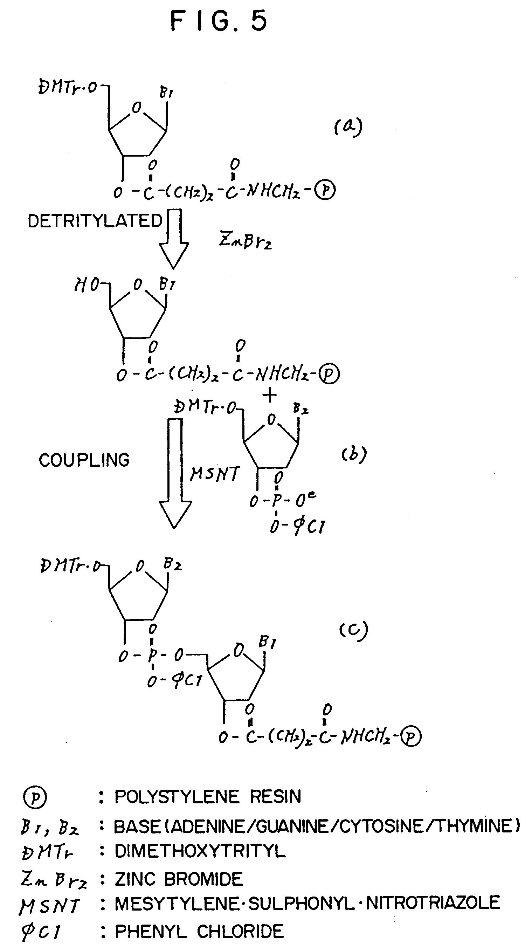

Fig. 1 shows the amino acid sequence of hEGF and the corresponding DNA sequence. Since the amino acid sequence of hEGF has been determined by H. Gregory et al., its structural gene may be deduced from the well known counterpart table of amino acid-genetic codon. However, a genetic codon is not decisively determined because degenerate codons usually exist. Therefore, genetic codon which appears in E. coli with high frequency was selected for every amino acid, based on the table showing frequency of appearance of genetic codon in each host [Ikemura, Cell Engineering, 2 (13), 78-89 (1983)]. Using thus determined structural gene of 159 nucleotides, a DNA sequence of 191 nucleotides containing linker for cloning them in plasmid and translation termination codon were designed. However, long chain DNA sequence as shown in Fig. 1 cannot be synthesized at once by a series of DNA chain extension manipulation (Fig. 5) according to the phosphoric acid triester method well known to be useful for DNA synthesis. That is, even if the yield for extension of one nucleotide is assumed to be 90%, 20 repetitions would result in the overall yield of about 12% and such yield would render purification difficult. Practically, the long chain double stranded DNA shown in Fig. 1 was divided into 28 single stranded oligonucleotide blocks (A-1 through A-14 and B-1 through B-14) as shown in Fig. 4 and each block was synthesized by DNA chain extension reaction according to the phosphoric acid triester method. Upon dividing into the blocks, the DNA sequence in each block was arranged most appropriately so as not to cause inappropriate interaction within and between the blocks and form gene having undesired structure.

Fig. 5 shows the procedure of the phosphoric acid triester method which comprises condensation-polymerizing mononucleotide (a) bound to polystyrene resin with another mononucleotide (b) to synthesize dinucleotide (c). In order to synthesize oligonucleotide composed of n number of nucleotides, this procedure may be repeated (n-1) times. The reaction was carried out in a conventional method.

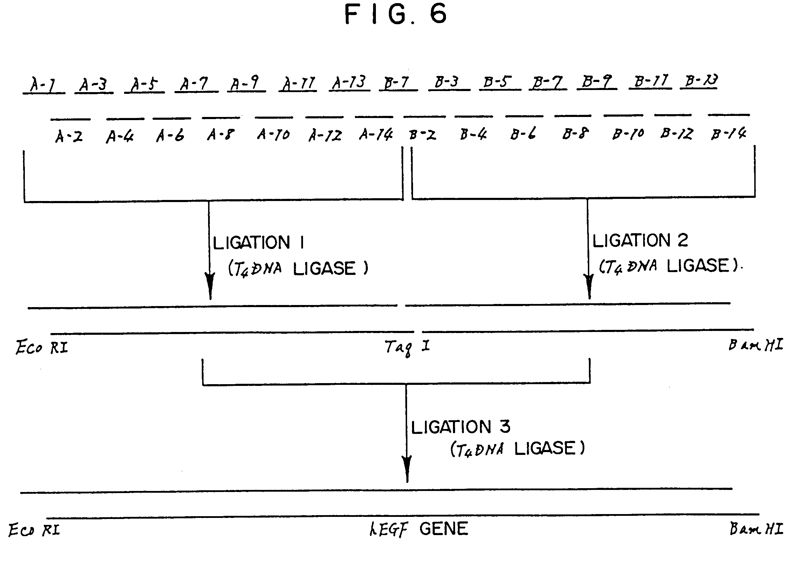

Fig. 6 shows the procedure for ligation of blocked oligonucleotides using T₄ DNA ligase. When the 28 blocks are ligated at once, there is a possibility that undesired combination might occur. Therefore, a two-step ligation method was adopted. The reaction was carried out in a conventional method.

The thus synthesized hEGF gene by organic chemistry technique was inserted into the cleaved portion caused by cleaving plasmid pBR322 with restriction enzymes Eco RI and Bam HI to give hEGF gene-bearing plasmid pBREGF [4.18 killo base pairs (Kb), Fig. 7]. E. coli was transformed with the thus obtained plasmid, whereby hEGF gene could be stably produced in large quantities.

### Example 2

Construction of plasmid pTREBT1 is described below (Fig. 11).

About 3 »g of pTRE2 and about 5 »g of pBREGF were cleaved with 56 U of restriction enzyme Eco RI and 60 U of Sal I followed by subjecting to agarose electrophoresis. 4.21 Kb (killo base pairs) DNA fragment containing trp promoter and 0.47 Kb DNA fragment containing hEGF gene were recovered from pTRE2 and pBREGF, respectively. After the recovered two DNA fragments were dissolved in TEN buffer (0.02 M Tris-HCl, 1 mM EDTA, 0.02 M NaCl, pH 8.0), 1/3 amount of the recovered hEGF DNA was added to 1/6 amount of the recovered trp promoter DNA. The DNA fragments were ligated with each other using 1500 U (20 »l in total) of T₄ DNA ligase. E. coli strain C600 was transformed with the ligated plasmid to give two transformants. The thus obtained transformants were cultured to obtain plasmid pTREBT1. E. coli strain HB101 was transformed with the thus obtained plasmid pTREBT1 to give E. coli HB101 [pTREBT1] bearing plasmid pTREBT1. The recombinant E. coli was deposited at the Fermentation Research Institute (FRI, located in Ibaragi Prefecture in Japan) under the Budapest Treaty and has received Accession No. FERM BP 2349.

### Example 3

Construction of plasmid pTRLBT1 is described below (Fig. 10).

pBREGF was cleaved with 16 U of each restriction enzymes Eco RI and Sal I followed by subjecting to polyacrylamide electrophoresis. Thus, 0.47 Kb DNA fragment containing hEGF gene was recovered. After purifying through treatment with phenol, DNA was dissolved in 20 »l of DNA ligase buffer (0.1 M Tris-HCl, 5 mM MgCl₂, pH 7.6). On the other hand, about 1 »g of pTRL 2 was cleaved with 8 U of each restriction enzymes Eco RI and Sal I followed by treatment with 1.2 U of bacterial alkaline phosphatase. Subsequently, treatment with phenol was repeated 3 times, and then dissolved in 20 »l of DNA ligase buffer. To 5 »l of hEGF DNA solution were added 2 »l of the pTRL2 solution described above and 30 »l of Solution A and 5 »l of Solution B of DNA Ligation Kit manufactured by Takara Shuzo Co., Ltd. to make the whole volume 42 »l. DNA ligation was performed at room temperature for 30 minutes. E. coli strain HB101 was transformed with the thus ligated plasmid to give more than 5,000 transformants. The thus obtained transformants were cultured and the resulting plasmid was cleaved with restriction enzyme. Examination of the resulting DNA fragments reveals that the plasmid is the desired pTRLBT1. Thus, plasmid pTRLBT1-bearing E. coli HB101 [pTRLBT1] could be obtained. The recombinant E. coli was deposited at FRI under the Budapest Treaty and has received Accession No. FERM BP 2348.

### Example 4

Production of hEGF in E. coli is described below.

E. coli HB101 [pTREBT1] and E. coli HB101 [pTRLBT1] were cultured under the following conditions, respectively, to produce hEGF.

One platinum loop of the stock strain, E. coli HB101 [pTREBT1] or E. coli HB101 [pTRLBT1], was inoculated on 10 ml of L medium (10 g of peptone, 5 g of yeast extract, 5 g of NaCl, 1 g of glucose, 50 mg of ampicillin, 1 liter of water; pH 7.2) followed by culturing at 37°C overnight. The preincubated solution 1 ml was taken and centrifuged at 15000 rpm for one minute to recover the cells. Then, the cells were washed with 1 ml of M9 medium (1 g of NH₄Cl, 6 g of Na₂HPO₄, 3 g of KH₂PO₄, 0.5 g of NaCl, 0.015 g of CaCl₂·2H₂O, 0.1 g of MgSO₄·7H₂O, 2.5 g of Casamino acid, 5 g of glucose, 0.1 g of thiamine, 50 mg of ampicillin, 1 liter of water; pH 7.4). The cells were resuspended in 1 ml of M9 medium. The whole amount of the suspension was inoculated on 10 ml of M9 medium followed by incubation at 37°C for 7 hours. In order to induce the expression of gene, 15 »g/ml of IA (3-β-indolylacrylic acid) was added thereto one hour after the incubation. After completion of the incubation, the cells were collected. After washing with 2 ml of 50 mM Tris-HCl buffer (pH 7.5), the cells were resuspended in 1 ml of the same buffer. To the cell suspension were added 18 »l of phenylmethylsulfonyl fluoride (at a concentration of 10 mg/ml), 10 »l of lysozyme (at a concentration of 10 mg/ml) and 20 »l of 0.25 mM EDTA. After agitation, the mixture was kept in ice water for 30 minutes. Then, the suspension was subjected to ultrasonication for 3 minutes to become transparent. To the solution was added 0.05 ml of 8 M urea solution. After shaking at 37°C for 60 minutes, the mixture was centrifuged at 30000 rpm for 30 minutes and the precipitates were removed. Subsequently, dialysis was performed 3 times against 50 mM Tris-HCl buffer and the dialysate was provided for radiorecepter assay (RRA).

RRA was carried out as follows. The hEGF activity was indicated in terms of mouse EGF standard weight required to exhibit the same activity. Labeled mouse EGF 0.1 ml or sample 0.1 ml and 0.1 ml of ¹³¹I-labeled mouse EGF were added to 0.2 ml of mouse microsome fraction. After agitation, the mixture was reacted at room temperature for 90 minutes. The reaction mixture was centrifuged at 4°C for 5 minutes at 60000 G. After the supernatant was removed by suction, radioactivity of the precipitates was measured. The hEGF activity per 1 ml of the culture solution thus measured is shown in Table 1.

**Table 1**

| Production of hEGF in E. coli | |
|---|---|
| E. coli | Amount Produced (ng/ml) |
| HB101 [pTREBT1] | 1100 |
| HB101 [pTRLBT1] | 900 |

### Example 5

Using E. coli HB101 [pTREBT1], fed-batch culture was carried out. The procedure and the results are shown below.

In a small scale fermentor of 5 liters, 1.8 liter of M9 culture medium (containing 1 g/l of glucose and 20 mg/l of tryptophan) was charged and 200 ml of the preincubation solution was inoculated to initiate culture. Fed-batch medium (200 g of glucose, 100 g of Casamino acid, 0.1 g of ampicillin, 1 liter of water; pH 7.0) was fed 1.5 hour after glucose had been consumed, using acetate concentration as a feeding index. That is, when the acetate concentration in the culture solution exceeded 2 g/l, the feeding rate was decreased and when the acetate concentration became 1 g/l or less, the feeding rate was increased. As the result, 6000 ng/ml of hEGF could be produced 8 hours after the incubation.

### Example 6

Synthesis of DNA fragment for construction of plasmid vectors pTRLBT2 and pTREBT2 is described below.

According to the present invention, DNA sequence of the linker region is primarily determined, since it is aimed to express matured hEGF, utilizing E. coli-derived trp P/O and translation regulatory region (SD sequence) of trpL or trp P/O and translation regulatory region (SD sequence) of trpE. That is, the start codon ATG of hEGF gene is arranged to locate at the start codon ATG site of trpL immediately downstream trp P/O. Alternatively, the start codon ATG is arranged to locate at the start codon ATG site of trpE. Utilizing the cleavage site of restriction enzyme Hinf I (Fig. 1) at the 15th nucleotide from ATG of hEGF gene so as to conform the already chemically synthesized hEGF gene cloned into plasmid pBREGF to its reading frame, 50 bp DNA fragment from the Hpa I site upstream trp P/O to the Hinf I site in the hEGF gene and 186 bp DNA fragment (C1 to C8) were synthesized. DNA sequences of these fragments are shown in Figs. 8 and 9. Synthesis procedures by organic chemistry were similarly performed as in the case of hEGF gene.

### Example 7

Construction of plasmid pTRLBT2 is explained below by referring to Fig. 12.

After about 30 »g of pTRLBT1 was cleaved with restriction enzymes Hpa I and Sph I, 0.8% agarose gel electrophoresis was performed to recover 4 Kb DNA fragment and 570 bp Hpa I/Sph I fragment containing hEGF gene to be used for constructing the vector pTRLBT2. After the latter fragment was further cleaved with restriction enzyme Hinf I, 6% acrylamide gel electrophoresis was conducted to give 340 bp Hinf I/Sph I fragment containing the most part of hEGF gene.

On the other hand, 50 bp Hpa I/Hinf I fragment (Fig. 8) containing trp P/O, translation regulatory gene of trpL and genetic codons corresponding to 3 amino acids at the N-terminal of hEGF was purified by 8M urea-containing 20% polyacrylamide gel electrophoresis. After recovering from the gel, the fragment was dissolved in DNA ligase buffer [100 mM Tris-HCl (pH 7.6), 5 mM MgCl₂]. The solution was once heated to 70°C and then gradually cooled to room temperature to effect annealing.

Then, 300 ng of the aforesaid 340 bp fragment and 100 ng of the 50 bp fragment dissolved in the reaction buffer [100 mM Tris-HCl (pH 7.6), 5 mM MgCl₂] were ligated with each other using 700 units of T₄ DNA ligase. In order to remove the self-ligated DNA fragment at the Sph I site, the DNA solution was treated with phenol to inactivate T₄ DNA ligase. Cleavage was again performed with restriction enzyme Sph I followed by subjecting to 5% acrylamide gel electrophoresis. Thus, 390 bp Hpa I/Sph I fragment containing trp P/O and entire hEGF gene starting from ATG was obtained.

Subsequently, T₄ polynucleotide kinase was reacted with this fragment to convert the Hpa I side into the phosphate, which was susceptible to cloning onto a vector. Then, the phosphated fragment was ligated with 400 ng of the vector (4 Kb) prepared by the above preparation method, using 500 units of T₄ DNA ligase. E. coli HB101 was transformed with the thus obtained DNA mixture to give plasmid pTRLBT2-bearing E. coli HB101 [pTRLBT2], which was deposited at FRI under the conditions of the Budapest Treaty and has received the accession number FERM BP 2351.

### Example 8

Construction of plasmid pTREBT2 which is a vector capable of expressing matured hEGF (Fig. 13) is described below. First, 340 bp Hinf I/Sph I fragment containing the most part of hEGF gene was obtained by the procedure described in Example 7.

On the other hand, 186 bp Hpa I/Hinf I fragment (Fig. 9) containing trp P/O, trpL structural gene and translation regulatory gene of trpE and genetic codons corresponding to 3 amino acids at the N-terminal of hEGF was purified by 8M urea-containing 20% polyacrylamide gel electrophoresis. After recovering from the gel, the fragment was dissolved in DNA ligase buffer [100 mM Tris-HCl (pH 7.6), 5 mM MgCl₂]. The solution was once heated to 70°C and then gradually cooled to room temperature to effect annealing.

Then, 300 ng of the aforesaid 340 bp fragment and 300 ng of the 186 bp fragment dissolved in the reaction buffer [100 mM Tris-HCl (pH 7.6), 5 mM MgCl₂] were ligated with each other using 700 units of T₄ DNA ligase. In order to remove the self-ligated DNA fragment at the Sph I site, the DNA solution was treated with phenol to inactivate T₄ DNA ligase. Cleavage was again performed with restriction enzyme Sph I followed by subjecting to 5% acrylamide gel electrophoresis. Thus, 526 bp Hpa I/Sph I fragment containing trp P/O and entire hEGF gene starting from ATG was obtained.

Next, T₄ polynucleotide kinase was reacted with this fragment to convert the Hpa I side into the phosphate, which was susceptible to cloning onto a vector. Then, the phosphated fragment was ligated with 400 ng of the vector (4 Kb) prepared in Example 7, using 500 units of T₄ DNA ligase. E. coli HB101 was transformed with the thus obtained DNA mixture to give plasmid pTREBT2-bearing E. coli HB101 [pTREBT2], deposited under the conditions of the Budapest Treaty with the accession number FERM BP 2350.

### Example 9

Production of hEGF in E. coli is described below.

One platinum loop of the stock strain, E. coli HB101 [pTRLBT2] or HB101 [pTREBT2], was inoculated on 3 ml of M9 medium (1 g of NH₄Cl, 6 g of Na₂HPO₄, 3 g of KH₂PO₄, 0.5 g of NaCl, 0.015 g of CaCl₂·2H₂O, 0.1 g of MgSO₄·7H₂O, 2.5 g of Casamino acid, 5 g of glucose, 0.1 g of thiamine, 1 g of proline, 50 mg of ampicillin, 1 liter of water; pH 7.4) followed by shake culture at 37°C overnight. Then 2 ml of the culture solution was inoculated on 38 ml of M9 medium having the composition described above followed by shake culture at 37°C for 4 hours. After completion of the incubation, the cells were collected, washed with 2 ml of 50 mM Tris-HCl buffer (pH 7.5) and suspended in 1 ml of the same buffer. To the cell suspension were added 18 »l of phenylmethylsulfonyl fluoride at a concentration of 10 mg/ml, 10 »l of lysozyme at a concentration of 10 mg/ml and 20 »l of 250 mM EDTA. After agitation, the mixture was kept in ice water for 30 minutes. Then, the suspension was subjected to ultrasonication for 3 minutes to homogenize the cells. Then 960 mg of urea and about 0.7 ml of 50 mM Tris-HCl buffer (pH 7.5) were added to make the whole volume 2 ml. After shaking at 37°C for one hour, the mixture was centrifuged at 70000 G for 30 minutes and the precipitates were removed. Subsequently, the supernatant containing denatured protein was dialyzed 3 times against 50 mM Tris-HCl buffer to remove urea, and then provided as a sample for radiorecepter assay (RRA).

RRA was carried out as follows.

To 0.2 ml of mouse liver microsome fragment were added 0.1 ml of standard EGF or 0.1 ml of a sample and 0.1 ml of ¹³¹I-labeled mouse EGF. After agitation, the mixture was reacted at room temperature for 90 minutes to complete competitive binding reaction of the standard EGF or sample with ¹³¹I-labeled mouse EGF for the EGF recepter. The reaction mixture was then centrifuged at 4°C for 5 minutes at 6000 G. After the supernatant was removed by suction, radioactivity of the ¹³¹I-labeled mouse EGF bound to the receptor contained in the precipitates was measured to quantitatively determine hEGF in the sample. Difference in specific activity between mouse EGF and hEGF was corrected; the hEGF activities per 1 ml of the culture solution of HB101 [pTRLBT2] and HB101 [pTREBT2] were both 24 ng.

While the invention has been described in detail and with reference to specific embodiments thereof, it will be apparent to one skilled in the art that various changes and modifications can be made therein without departing from the spirit and scope thereof.

## Claims

1. A process for the production of human epidermal growth factor comprising,
culturing recombinant E.coli bearing a plasmid vector containing a recombinant DNA sequence comprising a tryptophan regulatory gene, a gene coding for the N-terminal of trpL polypeptide in the tryptophan operon downstream said regulatory gene and a gene represented by formula (I) coding for 53 amino acids of human epidermal growth factor ligated with said gene coding for the N-terminal through a linker; and
recovering the human epidermal growth factor thus expressed.
Formula I

A A C T C C G A C T C T
G A A T G C C C G C T G T C C C A C G A T
G G T T A C T G C C T G C A C G A C G G C
G T T T G T A T G T A T A T C G A G G C G
C T G G A C A A A T A T G C G T G T A A C
T G T G T A G T A G G C T A C A T C G G C
G A A C G C T G C C A G T A C C G T G A C
C T G A A A T G G T G G G A A C T G C G C

2. A process for the production of human epidermal growth factor according to claim 1 comprising,
culturing recombinant E.coli bearing a plasmid vector containing a recombinant DNA sequence comprising a tryptophan regulatory gene, a gene coding for the N-terminal of trpE polypeptide in the tryptophan operon downstream said regulatory gene and a gene represented by formula (I) coding for 53 amino acids of human epidermal growth factor ligated with said gene coding for the N-terminal through a linker; and
recovering the human epidermal growth factor thus expressed.

3. A process according to Claim 1 or 2, wherein said recombinant E.coli is cultured in the presence of 3-β-indolylacrylic acid (IAA).

4. A process according to Claim 1 or 2, wherein said recombinant E.coli is cultured in a tryptophan-free medium.

5. A DNA sequence comprising a gene coding for the N-terminal of trpL polypeptide having ligated therewith a gene represented by formula (I) through a linker.

6. A DNA sequence comprising a gene coding for the N-terminal of trpE polypeptide having ligated therewith a gene represented by formula (I) through a linker.

7. A DNA sequence according to Claim 5 wherein a DNA sequence described below is ligated with a gene of formula (I).

A T G A A A G C A A T T T C G T G G A A
T T C G G A T C C A G G A A T C T A G A A
A T G

8. A DNA sequence according to claim 6, wherein a DNA sequence as described below is ligated with a gene of formula (I)

A T G C A A A C A C A A A A A C C G A C T
G G G A A T T C G G A T C C A G G A A T
C T A G A A A T G

9. A plasmid vector containing a tryptophan regulatory gene and a recombinant DNA sequence according to Claim 5.

10. A plasmid vector containing a tryptophan regulatory gene and a recombinant DNA sequence according to Claim 6.

11. A plasmid vector according to Claim 9, which is pTRLBT1 (FERM BP-2348).

12. A plasmid vector according to Claim 10, which is pTREBT1 (FERM BP-2349).

13. A recombinant E.coli bearing a plasmid vector according to Claims 9 or 10.

14. A recombinant E.coli according to Claim 13, which is [FERM BP 2348].

15. A recombinant E.coli according to Claim 13, which is [FERM BP 2349].

## Patentansprüche

1. Verfahren zur Herstellung von humanem Epidermis-Wachstumsfaktor, umfassend
Züchten von recombinantem E.coli, welches einen Plasmidvektor enthält, der eine recombinante DNA-Sequenz beinhaltet, die ein Tryptophan-Regulatorgen, ein den N-terminalen Bereich des trpL-Polypeptids in dem Tryptophan-Operon stromabwärts des Regulatorgens codierendes Gen und ein durch die Formel (I) dargestelltes, 53 Aminosäuren des humanen Epidermis-Wachstumsfaktor codierendes Gen, das mit dem den N-terminalen Bereich codierenden Gen durch einen Linker verknüpft ist, umfaßt, und
Gewinnen des so exprimierten, humanen Epidermis-Wachstumsfaktors.
Formel (I)

A A C T C C G A C T C T
G A A T G C C C G C T G T C C C A C G A T
G G T T A C T G C C T G C A C G A C G G C
G T T T G T A T G T A T A T C G A G G C G
C T G G A C A A A T A T G C G T G T A A C
T G T G T A G T A G G C T A C A T C G G C
G A A C G C T G C C A G T A C C G T G A C
C T G A A A T G G T G G G A A C T G C G C

2. Verfahren zur Herstellung von humanem Epidermis-Wachstumsfaktor nach Anspruch 1, umfassend
Züchten von recombinantem E.coli, welches einen Plasmidvektor enthält, der eine recombinante DNA-Sequenz beinhaltet, die ein Tryptophan-Regulatorgen, ein den N-terminalen Bereich des trpE-Polypeptids in dem Tryptophan-Operon stromabwärts des Regulatorgens codierendes Gen und ein durch die Formel (I) dargestelltes, 53 Aminosäuren des humanen Epidermis-Wachstumsfaktor codierendes Gen, das mit dem den N-terminalen Bereich codierenden Gen durch einen Linker verknüpft ist, umfaßt, und
Gewinnen des so exprimierten, humanen Epidermis-Wachstumsfaktors.

3. Verfahren nach Anspruch 1 oder 2, worin das recombinante E.coli in Anwesenheit von 3-β-Indolylacrylsäure (IAA) gezüchtet wird.

4. Verfahren nach Anspruch 1 oder 2, worin das recombinante E.coli in einem Tryptophan-freien Medium gezüchtet wird.

5. DNA-Sequenz, die ein den N-terminalen Bereich des trpL-Polypeptids codierendes Gen umfaßt, das mit einem durch die Formel (I) dargestellten Gen durch einen Linker verknüpft ist.

6. DNA-Sequenz, die ein den N-terminalen Bereich des trpE-Polypeptids codierendes Gen umfaßt, das mit einem durch die Formel (I) dargestellten Gen durch einen Linker verknüpft ist.

7. DNA-Sequenz nach Anspruch 5, worin eine nachstehend beschriebene DNA-Sequenz mit einem Gen der Formel (I) ligiert ist.

A T G A A A G C A A T T T T C G T G G A A
T T C G G A T C C A G G A A T C T A G A A
A T G

8. DNA-Sequenz nach Anspruch 6, worin eine nachstehend beschriebene DNA-Sequenz mit einem Gen der Formel (I) verknüpft ist.

A T G C A A A C A C A A A A A C C G A C T
G G G G A A T T C G G A T C C A G G A A T
C T A G A A A T G

9. Plasmidvektor, der ein Tryptophan-Regulatorgen und eine recombinante DNA nach Anspruch 5 enthält.

10. Plasmidvektor, der ein Tryptophan-Regulatorgen und eine recombinante DNA nach Anspruch 6 enthält.

11. Plasmidvektor nach Anspruch 9, der pTRLBT1 (FERM BP-2348) ist.

12. Plasmidvektor nach Anspruch 10, der pTREBT1 (FRM BP-2349) ist.

13. Recombinantes E.coli, das einen Plasmidvektor gemäß den Ansprüchen 9 oder 10 enthält.

14. Recombinantes E.coli nach Anspruch 13, das [FERM BP 2348] ist.

15. Recombinates E.coli nach Anspruch 13, das [FERM BP 2349] ist.

## Revendications

1. Procédé de production du facteur de croissance épidermique humain dans lequel
on cultive un E. coli recombinant portant un vecteur plasmidique contenant une séquence d'ADN recombinant comprenant un gène régulateur de tryptophane, un gène codant pour l'extrémité N-terminale du polypeptide trpL dans l'opéron tryptophane en aval dudit gène régulateur et un gène représenté par la formule (I) codant pour 53 acides aminés du facteur de croissance épidermique humain ligaturé avec ledit gène codant pour l'extrémité N-terminale par l'intermédiaire d'un lieur; et
on recueille le facteur de croissance épidermique humain ainsi exprimé.
Formule I

A A C T C C G A C T C T
G A A T G C C C G C T G T C C C A C G A T
G G T T A C T G C C T G C A C G A C G G C
G T T T G T A T G T A T A T C G A G G C G
C T G G A C A A A T A T G C G T G T A A C
T G T G T A G T A G G C T A C A T C G G C
G A A C G C T G C C A G T A C C G T G A C
C T G A A A T G G T G G A A C T G C G C

2. Procédé de production du facteur de croissance épidermique humain selon la revendication 1, dans lequel on cultive un E. coli recombinant portant un vecteur plasmidique contenant une séquence d'ADN recombinant comprenant un gène régulateur de tryptophane, un gène codant pour l'extrémité N-terminale du polypeptide trpE dans l'opéron tryptophane en aval dudit gène régulateur et un gène représenté par la formule (I) codant pour 53 acides aminés du facteur de croissance épidermique humain ligaturé avec ledit gène codant pour l'extrémité N-terminale par l'intermédiaire d'un lieur; et
on recueille le facteur de croissance épidermique humain ainsi exprimé.

3. Procédé selon la revendication 1 ou 2, dans lequel on cultive ledit E. coli recombinant en présence d'acide 3-β-indolyl-acrylique (IAA).

4. Procédé selon la revendication 1 ou 2, dans lequel on cultive ledit E. coli recombinant dans un milieu dépourvu de tryptophane.

5. Séquence d'ADN comprenant un gène codant pour l'extrémité N-terminale du polypeptide trpL auquel est ligaturé un gène représenté par la formule (I) par l'intermédiaire d'un lieur.

6. Séquence d'ADN comprenant un gène codant pour l'extrémité N-terminale du polypeptide trpE auquel est ligaturé un gène représenté par la formule (I) par l'intermédiaire d'un lieur.

7. Séquence d'ADN selon la revendication 5, dans laquelle une séquence d'ADN décrite ci-dessous est ligaturée avec un gène de formule (I).

A T G A A A G C A A T T T T C G T G G A A

T T C G G A T C C A G G [?] A T C T A G A A

A T G

8. Séquence d'ADN selon la revendication 6, dans laquelle une séquence d'ADN telle que décrite cidessous est ligaturée avec un gène de formule

A T G C A A A C A C A A A A A C C G A C T
G G G G A A T T C G G A T C C A G G A A T
C T A G A A A T G

9. Vecteur plasmidique contenant un gène régulateur de tryptophane et une séquence d'ADN recombinant selon la revendication 5.

10. Vecteur plasmidique contenant un gène régulateur de tryptophane et une séquence d'ADN recombinant selon la revendication 6.

11. Vecteur plasmidique selon la revendication 9, qui est pTRLBT1 (FERM BP-2348).

12. Vecteur plasmidique selon la revendication 10, qui est pTREBT1 (FERM BP-2349).

13. E. coli recombinant portant un vecteur plasmidique selon la revendication 8 ou 9.

14. E. coli recombinant selon la revendication 13, qui est (FERM BP-2348).

15. E. coli recombinant selon la revendication 13, qui est (FERM BP-2349).
